Europäisches Patentamt

European Patent Office

Office européen des brevets

(11) Numéro de publication: **0 261 512 B1**

(12) **FASCICULE DE BREVET EUROPEEN**

(45) Date de publication de fascicule du brevet: **08.07.92**  (51) Int. Cl.⁵: **A61F 13/00**

(21) Numéro de dépôt: **87113276.7**

(22) Date de dépôt: **11.09.87**

(54) **Tissu élastique perfectionné.**

(30) Priorité: **18.09.86 CH 3742/86**

(43) Date de publication de la demande:
**30.03.88 Bulletin 88/13**

(45) Mention de la délivrance du brevet:
**08.07.92 Bulletin 92/28**

(84) Etats contractants désignés:
**ES GB IT SE**

(56) Documents cités:
**EP-A- 0 120 117**
**US-A- 3 371 668**
**US-A- 4 515 594**

(73) Titulaire: **Vitapharm Basel AG**
**Kannenfeldstrasse 56**
**CH-4012 Basel(CH)**

(72) Inventeur: **Aichele, Dieter A.**
**Spechtweg 8**
**CH-4125 Riehen(CH)**

(74) Mandataire: **Frossard, Michel, Dr. et al**
**A. Braun, Braun, Héritier, Eschmann AG, Patentanwälte Holbeinstrasse 36-38**
**CH-4051 Basel(CH)**

Rank Xerox (UK) Business Services

## Description

La présente invention se réfère à un tissu élastique, utilisables notamment pour des bandages et des pansements, conforme au préambule de la revendication 1. Un tel tissu est connu du brevet US-A-3 371 668.

Les tissus élastiques destinés à l'application directe sur la peau doivent présenter une surface dont les propriétés ne soient pas irritantes. De même les vêtements confectionnés avec ces tissus doivent être exempts de coutures, d'ourlets, de bordures etc., qui produisent des irrégularités en cas de pression et sur leur surface. Il est également important que les matières utilisées pour la confection de tels vêtements soient bien tolérées par l'organisme humain, résistantes au lavage, sans la moindre agressivité et adaptables anatomiquement.

Actuellement, on essaie de réaliser les objectifs ci-dessus mentionnés au moyen du surfaçage du tissu élastique, ce qui provoque uniquement la diminution de son épaisseur. Pour compenser les inconvénients d'un tissu peu épais, habituellement on l'apprête, mais ceci ne fait qu'ajouter un inconvénient de plus au tissu étant donné que l'apprêt imperméabilise et qu'il s'agit d'un additif chimique;de plus, l'effet n'est que de courte durée.

A tout ceci s'ajoute le fait que les coutures ou jointures dans un tissu très fin s'avèrent toujours difficiles; de plus, si l'on utilise des fils élastiques de petit calibre ils doivent être tissés très tendus pour obtenir la même pression, ce qui limite le pourcentage possible d'élasticité et la propre durée du tissu.

Tenant compte de ce qui précède et afin de surmonter les inconvénients et problèmes que présentent les tissus actuels destinés aux bandages et articles cliniques, le but de la présente invention est d'obtenir un tissu ayant une surface d'un toucher agréable, qui peut se combiner avec des fermetures autoadhésives, dont la confection n'exige pas d'ourlets aux extrémités ou aux bordures, qui laisse passer la transpiration et qui présente une élasticité latérale. Ce but est atteint par un tissu conforme à la revendication 1.

Un tissu présentant ces propriétés est bien approprié à la fabrication de bandes élastiques, susceptibles de recevoir à l'une ou aux deux extrémités des fermetures de type adhésif, ce qui favorise son application car le relachement ou une chute possible sont ainsi prévenus.

A partir du tissu objet de l'invention, on peut confectionner des articles tels que gantelets ou chevillères, qui s'adaptent anatomiquement et qui trouvent des applications en thermothérapie et pressothérapie. Ces articles, renforcés par des rubans de matériel adhésif et couverts pour plus de sécurité avec une bande, fournissent des bandages transformables pour un degré plus ou moins grand d'immobilisation ou correction.

On peut donc en confectionner, notamment, des gaines de hanches pour usage postopératoire, des gaines pour usage postopératoire après liposuccion (chirurgie esthétique); des soutiens-gorge pour usage postopératoire en chirurgie esthétique; des bandages compressifs pour brûlés, amputations, applications angiologiques (oedèmes variqueux, etc.); des serviettes élastiques pour sportifs, des bandes pour poignets, des bandes pour la tête, etc., des gaines pour nouveaux-nés et autres articles qui requièrent un contact direct du tissu avec la peau.

De manière générale, les tissus élastiques perfectionnés selon l'invention comprennent une pièce de tissu, un ourdissage élastique, une autre pièce du même ou d'un autre tissu, le tout uni par une chaînette ou couture élastique qui unit les deux pièces de tissu et enveloppe en même temps les fils élastiques. Ce procédé se réalise avec les fils élastiques en tension qui est très supérieure à celle qu'ont ou celle que pourraient avoir les deux ou trois tissus mis en jeu.

Le résultat est un tissu élastique obtenu par la couture de deux tissus plus ou moins élastiques ou totalement rigides avec des surpiqûres élastiques, qui ont une âme, un bourrage élastique qui le force, le resserre et le fronce, les deux surfaces rugueuses étant maintenues en repos, toujours souples, l'une d'elles pouvant être de polyamide ce qui la rend apte à être associée à une fermeture de type adhésif.

On obtient donc un tissu dont l'une des faces ou les deux sont aptes à recevoir une fermeture adhésive et sont très agréable à la peau. La surface en contact avec la peau peut être en polyamide, mais elle peut être également en coton bouclé ou frisé, on peut également appliquer un tissu sur une des faces, insérant ou situant du fil à sa place, réalisant un fini brossé à cette surface de fil approprié, acryle, laine, etc. Le résultat est une face brossée, plus ou moins velue comme une couverture; l'autre surface peut être munie d'une fermeture de type adhésif, en coton, coton éponge ou tout autre matériel textile, comme la laine ou tous les mélanges qui peuvent se faire.

Afin de faciliter la compréhension de l'invention, une planche de dessins où est représenté un cas de réalisation cité à titre d'exemple est jointe au présent mémoire descriptif.

Dans les dessins:

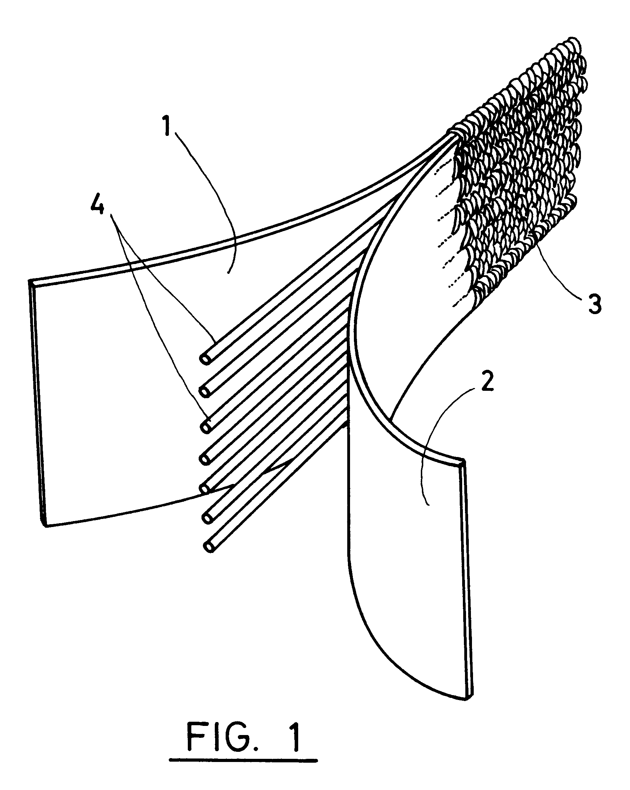

La figure 1 montre en perspective les composants du bandage.

La figure 2 représente l'utilisation du bandage, en combinaison avec une chevillère, pour obtenir un bandage transformable pour une plus ou moins grande immobilisation ou correction.

En se référant aux figures, on voit dans leur réalisation un bandage élastique qui comprend une première pièce de tissu 1, une second pièce 2 du même ou d'un autre tissu, le tout uni par une "chaînette" ou couture élastique 3, qui associe les deux pièces de tissu 1 et 2 et enveloppe en même temps les fils élastiques 4, de tension supérieure à celle qu'ont ou celle que pourraient avoir les deux ou trois tissus qui sont mis en jeu.

Une ou les deux faces du tissu peuvent être aptes à être munies d'une fermeture autoadhésive. De même, les composants du bandage peuvent être les deux en polyamide, en acryle et polyamide, ou en coton et polyamide.

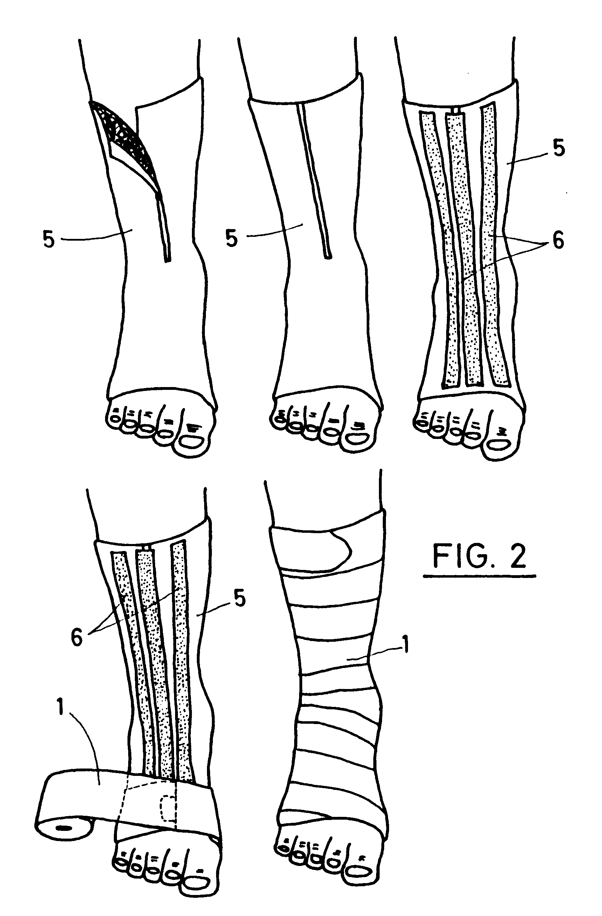

Dans la figure 2 est représenté un cas pratique d'utilisation du bandage en combinaison avec une chevillère 5, à laquelle ont été appliqués des rubans de matériel autoadhésif 6 qui sont couverts par une bande du type décrit, ce qui fournit des bandages transformables pour un plus ou moins grand degré d'immobilisation.

L'invention, dans son essence, peut être mise en pratique sous d'autres formes de réalisation qui diffèrent dans le détail de celle indiquée ci-dessus à titre d'exemple et qui entrent également dans le cadre de l'invention. Elle pourra être construite en toutes formes et dimensions avec les matériaux les plus appropriés, le tout restant compris dans d'esprit des revendications.

## Revendications

1.  Tissu élastique, utilisable notamment pour des bandages et pansements, comprenant une première bande de tissu (1) et une seconde bande (2) du même ou d'un autre tissu, les deux bandes de tissu étant placées face à face dans le sens de la lonqueur et comprenant entre elle une rangée de fils élastiques (4) placés parallèlement dans le sens longitudinal et soumis à une tension supérieure à celle des bandes de tissu, caractérisé en ce que l'un et l'autre des tissus de la première et seconde bande sont en un matériel agréable au toucher, perméable à la transpiration et présentant une élasticité latérale, et que l'ensemble est uni par une série de piqûres (3) formant trame, allant d'un bord à l'autre dans le sens transversal et passant de part et d'autre des fils élastiques de la face extérieure de l'un à la face extérieure de l'autre tissu, de sorte que la surface des tissus au repos soit froncée.

2.  Tissu élastique selon la revendication 1, caractérisé en ce que l'une des bandes (1, 2) de tissu ou les deux sont en polyamide ou en coton bouclé.

3.  Tissu élastique selon la revendication 2, caractérisé en ce que l'une des bandes (1, 2) de tissu est en polyamide et comporte à l'une de ses extrémités une fermeture de type autoadhésif.

4.  Tissu élastique selon l'une des revendication 1 à 3, caractérisé en ce qu'il comporte en outre, sur l'une des faces extérieures, une troisième bande du même ou d'un autre tissu unie à l'ensemble par une série de piqûres formant trame comme décrit à la revendication 1.

## Claims

1.  Elastic fabric, which can be used, in particular, for bandages and dressings, comprising a first strip of fabric (1) and a second strip of fabric (2) made of the same or a different fabric, the two strips of fabric being placed against each other in the lengthwise direction and comprising, between them, a row of elastic threads (4) positioned parallel to each other in the longitudinal direction and subjected to a tension which is greater than that of the fabric strips, characterized in that the fabrics of both strips are made of a material which is pleasant to touch, permeable to sweat and having a sideways elasticity and that the whole is joined together by a series of stitches (3) forming a weft, going from one edge to the other in the transverse direction and passing on either side of the elastic threads from the outer face of one to the outer face of the other fabric so that the surface of the fabric at rest is shirred.

2.  Elastic fabric according to Claim 1, characterized in that one or both of the fabric strips (1, 2) are made of polyamide or of looped cotton.

3.  Elastic fabric according to Claim 2, characterized in that one of the fabric strips (1, 2) is made of polyamide and comprises, at one of its ends, a self-adhesive type closure.

4.  Elastic fabric according to one of Claims 1 to 3, characterized in that it additionally comprises, on one of the outer surfaces, a third strip of the same or a different fabric joined to the whole by a series of stitches forming a weft as described in Claim 1.

## Patentansprüche

1.  Elastisches Gewebe, welches insbesondere für Bandagen und Verbände brauchbar ist und einen ersten Gewebestreifen (1) und einen zweiten Streifen (2) aus demselben oder einem anderen Gewebe umfasst, wobei beide Gewebestreifen in der Längsrichtung einander gegenüberliegen und dazwischen eine Reihe von elastischen Fäden (4), die in der Längsrichtung zueinander parallel liegen und einer höheren Spannung als jene der Gewebestreifen unterliegen, umschliessen, dadurch gekennzeichnet, dass die Gewebe des ersten und des zweiten Gewebestreifens aus angenehm anzufühlendem, schweissdurchlässigem und seitliche Elastizität aufweisendem Material bestehen, und dass das Ganze durch eine Reihe von Stichen (3) verbunden ist, welche den Schuss bilden, in der Querrichtung von Rand zu Rand und von der äusseren Fläche des einen Gewebes zur äusseren Fläche des anderen Gewebes zu beiden Seiten der elastischen Fäden verlaufen, so dass die Oberfläche des Gewebes im Ruhezustand gekräuselt ist.

2. Elastisches Gewebe nach Anspruch 1, dadurch gekennzeichnet, dass einer der Gewebestreifen (1, 2) oder beide aus Polyamid oder Bouclébaumwolle bestehen.

3. Elastisches Gewebe nach Anspruch 2, dadurch gekennzeichnet, dass einer der Gewebestreifen (1, 2) aus Polyamid besteht und an einem Ende einen Verschluss von selbstklebendem Typus umfasst.

4. Elastisches Gewebe nach einem der Ansprüche 1 bis 3, dadurch gekennzeichnet, dass es ausserdem auf einer der äusseren Flächen einen dritten Streifen aus demselben oder einem anderen Gewebe umfasst, welcher mit dem Ganzen durch eine Reihe von den Schuss bildenden Stichen, wie im Anspruch 1 beschrieben wird, verbunden ist.

FIG. 1

FIG. 2